# DEMANDE DE BREVET EUROPEEN

(11) **EP 3 132 758 A1**
(43) Date de publication de la demande: **22.02.2017**
(21) Numéro de dépôt: 16183028.6
(22) Date de dépôt: 05.08.2016
(51) Int. Cl.: A61B 17/17, A61B 17/80

(54) **SABOT D'AIDE AU POSITIONNEMENT ET A LA FIXATION D'UN IMPLANT CHIRURGICAL**

(30) Priorité: 19.08.2015 FR 1557811
(71) Demandeur: In2Bones, 69130 Ecully (FR)
(72) Inventeur: GAUNEAU, Bertrand, Xavier, François, 69570 DARDILLY (FR); FOURCAULT, Eric, Stéphane, 69130 ECULLY (FR); GIET, Jean-Christophe, Alain, 69006 LYON (FR)
(74) Mandataire: Weber, Jean-François

(57) **Abrégé**

- L'invention concerne un sabot (1) d'aide au positionnement et à la fixation d'un implant chirurgical destiné à être rapporté sur des corps osseux d'un patient, par exemple de son avant-bras, ledit sabot (1) comportant un corps principal (24) et étant caractérisé en ce que ledit corps principal (24) comporte :
- au moins une zone radio-transparente (25) et au moins une zone radio-opaque (26), et
- des moyens de positionnement (27) dudit sabot (1) relativement à l'implant chirurgical dans une position relative prédéterminée,
- de sorte à permettre le contrôle du positionnement dudit implant relativement auxdits corps osseux du patient.

- Dispositifs d'aide à la mise en place d'implants chirurgicaux d'ostéosynthèse.

## Description

La présente invention concerne de manière générale le domaine des dispositifs d'aide à la mise en place d'implants chirurgicaux d'ostéosynthèse, en particulier conçus pour le traitement de fractures ou de fêlures de corps osseux situés notamment dans l'avant-bras.

La présente invention concerne plus particulièrement un sabot d'aide au positionnement et à la fixation d'un implant chirurgical destiné à être rapporté sur des corps osseux d'un patient, par exemple de son avant-bras, ledit sabot comportant un corps principal.

La présente invention concerne également un kit chirurgical incluant un tel sabot.

Pour améliorer la reconstruction des os, par exemple des os de l'avant-bras d'un patient à la suite de certains types de fractures ou de fêlures de l'extrémité distale du radius, il est connu d'avoir recours à un implant chirurgical, généralement désigné dans le cas du traitement d'un radius, par le terme de « *plaque radiale* ». Ce type d'implant chirurgical est le plus souvent, voire exclusivement, formé par une plaque courbée constituée de métal (Ti6Al4V, CrCo, etc.) afin que la surface antérieure de la plaque soit de forme convexe pour épouser la forme de la portion extrémale du radius à réparer. La plaque radiale est destinée à être fixée par vissage sur au moins deux parties du radius séparées par fracture de manière à ce que ladite plaque enjambe le ou les traits de fracture et immobilise lesdites au moins deux parties du radius les unes par rapport aux autre pour favoriser leur fusion.

Lorsqu'il procède, au cours d'une opération chirurgicale, au positionnement de l'implant en regard des corps osseux à fusionner, le chirurgien doit prêter attention à ce que ledit implant soit parfaitement positionné en regard du ou des traits de fracture.

De plus, il doit particulièrement veiller à ce que les vis, une fois implantées, ne soient pas trans-articulaires, c'est-à-dire qu'elles ne viennent pas déboucher de la face opposée du corps osseux dans lequel elles sont fixées, au risque que lesdites vis viennent perturber le bon fonctionnement de l'articulation de l'os relativement aux os environnants.

Du fait de la très grande variété des morphologies du radius des patients, ces derniers sont susceptibles, dans certains cas, de subir des douleurs au cours du processus d'ostéosynthèse. Quelque fois, le frottement des tissus sur l'implant et notamment sur les arêtes de ce dernier, peut également être source de douleur, en dépit de la faible épaisseur de ce dernier.

En particulier, dans l'exemple considéré du traitement de fractures ou de fêlures de l'extrémité distale du radius, le chirurgien doit également être particulièrement vigilant quant au positionnement de la plaque d'ostéosynthèse en regard d'une zone anatomique très spécifique du radius, connue dans la profession sous le nom de ligne « *watershed ».*

Cette ligne « *watershed* » peut, en substance, être considérée comme la ligne de crête de la partie palmaire du radius, de part et d'autre de laquelle l'os est plus de bas, et sur laquelle les tendons viennent frotter en tension.

Il a été observé que lorsque la plaque d'ostéosynthèse vient malencontreusement à être positionnée de telle sorte qu'elle se projette au-delà de ladite ligne « *watershed* », cela provoque des irritations problématiques des tendons fléchisseurs environnants. Il est par conséquent fortement conseillé aux chirurgiens de veiller à la précision du positionnement de la plaque d'ostéosynthèse de sorte à éviter que celle-ci ne vienne déborder de cette ligne « *watershed ».*

Classiquement, le chirurgien en charge de la pose d'une telle plaque, positionne dans tout d'abord manuellement, avec ou sans outil particulier, cette dernière relativement au trait ou à la zone de fracture.

Puis, pour assurer la bonne orientation des vis de fixation de ladite plaque à travers les corps osseux, le chirurgien fait usuellement usage d'un dispositif, appelé sabot, qu'il vient placer en contact avec la surface libre de la plaque.

Un tel sabot présente en effet des trous oblongs, destinés à être placés selon une position prédéterminée en regard des perçages de fixation de la plaque. Ces trous oblongs sont configurés de telle sorte à permettre, dans un premier temps une limitation angulaire du positionnement d'un guide de perçage de l'os, puis dans un second temps, une fois l'os ainsi percé selon une orientation souhaitée, à permettre de guider la mise en place des vis de fixation de la plaque sur les corps osseux afin de permettre une mise en position aisée et précise de ces vis de fixation selon ladite orientation souhaitée.

Le chirurgien contrôle ensuite par radiographie, et ajuste au besoin itérativement, la position de ladite plaque relativement à la ligne « *watershed* ».

Une fois la plaque positionnée et solidarisée aux corps osseux par les vis de fixation mises en place grâce au sabot, le chirurgien contrôle également la bonne configuration desdites vis grâce à la prise de clichés radiographiques.

Enfin, le chirurgien démonte le sabot de la plaque et l'extrait hors de corps du patient, ledit sabot n'étant pas destiné à y rester implanté.

La composition métallique des plaques radiales connues, ainsi par ailleurs de celle des vis de fixation classiquement utilisées, les rend en effet radio-opaques, c'est-à-dire visible aux rayons X par contraste avec l'environnement biologique dans lequel elles sont implantées. Dès lors, le contrôle du positionnement de la plaque implantée relativement à la ligne « *watershed »* ainsi que le contrôle de l'orientation des vis de fixations est ainsi facilité.

Si les solutions connues mettant en oeuvre des plaques d'ostéosynthèse métalliques donnent généralement satisfaction, il est à noter que la radio-opacité de ces dernières empêche toutefois non seulement la visualisation des traits de fractures une fois la plaque implantée dans le patient, et ne permet en outre la visualisation des vis de fixation aux corps osseux considérés qu'uniquement par le biais de radiographies de profil.

Par conséquent, il a semblé que ces dites plaques d'ostéosynthèse pouvaient être améliorées et c'est pourquoi les inventeurs de la présente invention ont eux-mêmes, par ailleurs, récemment proposé un nouvel implant d'ostéosynthèse qui présente, entre autres avantages, une caractéristique de radio-transparence permettant de répondre efficacement aux limitations précitées.

Toutefois, il a été observé qu'un tel implant d'ostéosynthèse, aussi avantageux qu'il soit en regard des problématiques précédemment énumérées, n'offre pas au chirurgien de possibilité de contrôle précis, autre que visuel, du positionnement dudit implant en regard d'une zone anatomique donnée, par exemple la ligne « *watershed* » d'un radius.

En particulier, le chirurgien ne peut contrôler ce positionnement relatif à l'aide d'une prise classique de cliché radiographique puisque l'implant d'ostéosynthèse n'est pas visible à la radiographie.

L'invention vise en conséquence à porter remède à cet inconvénient technique, et à proposer un nouveau sabot d'aide au positionnement et à la fixation d'un implant chirurgical apte à être aisément rapporté sur ledit implant de sorte à permettre de manière pratique le contrôle précis de la position dudit implant relativement auxdits corps osseux du patient quand bien même ledit implant n'est pas lui-même directement visible par radiographie.

Un autre objet de l'invention vise à proposer un nouveau sabot et un nouveau kit chirurgical associé permettant de faciliter l'opération de positionnement et de fixation de l'implant dans le corps du patient, grâce à une meilleure appréciation de la position de l'implant chirurgical.

Un autre objet de l'invention vise à proposer un nouveau sabot et un nouveau kit chirurgical associé permettant un meilleur suivi de l'évolution de la fracture d'un os d'un patient.

Un autre objet de l'invention vise à proposer un nouveau sabot et un nouveau kit chirurgical associé rapide et pratique à mettre en oeuvre dans le cadre d'une opération chirurgicale.

Un autre objet de l'invention vise à proposer un nouveau sabot et un nouveau kit chirurgical associé dont la conception et la fabrication sont relativement aisées et peu coûteuses.

Un autre objet de l'invention vise à proposer un nouveau sabot et un nouveau kit chirurgical associé permettant de limiter les complications post-opératoires et la douleur subie par le patient.

Les objets assignés à l'invention sont atteints à l'aide d'un sabot d'aide au positionnement et à la fixation d'un implant chirurgical destiné à être rapporté sur des corps osseux d'un patient, par exemple de son avant-bras, ledit sabot comportant un corps principal et étant caractérisé en ce que ledit corps principal comporte :
- au moins une zone radio-transparente et au moins une zone radio-opaque, et
- des moyens de positionnement dudit sabot relativement à l'implant chirurgical dans une position relative prédéterminée,
de sorte à permettre le contrôle du positionnement dudit implant relativement auxdits corps osseux du patient.

Les objets assignés à l'invention sont également atteints à l'aide d'un kit chirurgical incluant un implant chirurgical destiné à être rapporté sur des corps osseux d'un patient, par exemple de son avant-bras, ainsi qu'un sabot conforme à la description ci-dessus.

D'autres objets et avantages de l'invention apparaîtront plus en détail à la lecture de la description qui suit, ainsi qu'à l'aide des dessins annexés fournis à titre purement explicatif et non limitatif, dans lesquels :
- La figure 1 illustre, selon une vue générale en perspective, un sabot d'aide au positionnement et à la fixation d'un implant chirurgical selon l'invention ;
- La figure 2 illustre, selon une vue longitudinale en perspective, un mode de réalisation préférentiel d'un implant d'ostéosynthèse apte à coopérer avec le sabot de l'invention ;
- La figure 3 illustre, selon une vue générale en perspective, le sabot de la figure 1, rapporté et fixé sur un implant d'ostéosynthèse, et associé avec un guide de perçage en vue de préparer la fixation dudit implant d'ostéosynthèse sur des corps osseux d'un patient ;
- La figure 4 illustre, selon une vue latérale, l'assemblage général d'un sabot selon l'invention, représenté de manière très schématique, sur un implant d'ostéosynthèse, elle-même rapportée et maintenue fixée sur des corps osseux au niveau d'une zone de fracture à l'aide de vis de fixations ;
- La figure 5 illustre, selon une vue de face, le sabot de la figure 1, rapporté et fixé sur un implant d'ostéosynthèse, l'ensemble étant positionné sur un radius distal en regard de la ligne « *watershed »* de ce dernier ;
- La figure 6 représente, selon une vue de dessus, le sabot de la figure 1 associé à un implant d'ostéosynthèse, pour former un kit chirurgical conforme à l'invention.

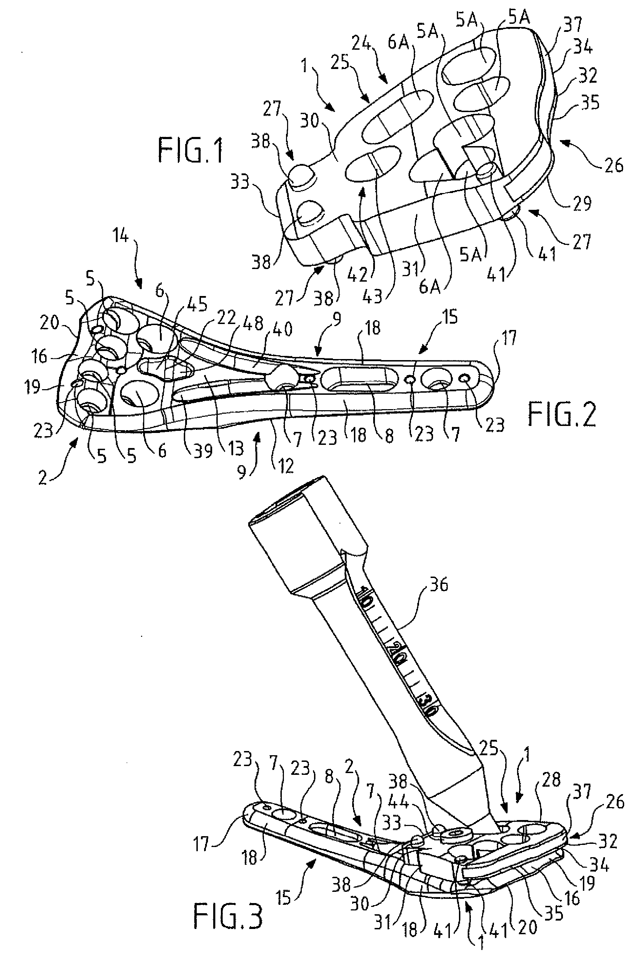

L'invention concerne en tant que tel un sabot 1 d'aide au positionnement et à la fixation d'un implant chirurgical 2 destiné à être rapporté sur des corps osseux 3, 4 d'un patient, sabot 1 dont un exemple de réalisation non limitatif et non exhaustif est illustré à la figure 1.

Ledit implant chirurgical 2 est, par exemple, destiné à être rapporté sur des corps osseux d'un avant-bras et/ou au poignet d'un patient. Dans cet exemple, ledit implant chirurgical 2 forme donc un implant d'avant-bras, en l'espèce une plaque radiale distale pour le traitement d'une fracture de l'extrémité distale du radius de l'avant-bras d'un patient.

L'invention ne se limite toutefois pas à un sabot 1 d'aide au positionnement et à la fixation d'un implant d'avant-bras, et pourra concerner par exemple un implant tibial ou un implant palmaire.

En tout état de cause, l'implant chirurgical 2, dont le sabot 1 selon l'invention vient aider au positionnement et à la fixation, est prévu pour être implanté dans le corps du patient au cours d'une opération chirurgicale effectuée par exemple sous anesthésie, en particulier à la suite d'une lésion d'un os du patient du type fracture ou fêlure osseuse, pour soigner ou contribuer à soigner ladite lésion au cours d'un processus d'ostéosynthèse. Le patient désigné par l'invention est de préférence un être humain, étant entendu que rien ne s'oppose à l'introduction et à la fixation dudit implant 2 à l'aide du sabot 1 de l'invention dans le corps d'un animal pour soigner des pathologies vétérinaires similaires correspondant aux pathologies humaines présentement décrites.

L'implant chirurgical 2, au positionnement et à la fixation duquel le sabot 1 de l'invention vient en aide, est destiné à être rapporté sur des corps osseux 3, 4 d'un patient, par exemple de son avant-bras et/ou de son poignet, par l'intermédiaire de moyens de fixation 5, 6, 7, 8 afin de maintenir en position lesdits corps osseux 3, 4 les uns par rapport aux autres, tel qu'illustré par exemple à la figure 2, afin en particulier de permettre, ou pour le moins de favoriser, l'ostéosynthèse de ces derniers.

L'implant chirurgical 2 est ainsi conçu pour être introduit dans le corps d'un patient, et pour être fixé temporairement ou définitivement aux corps osseux 3, 4 lésés. On entend par « *moyens de fixation* », des éléments appartenant ou non à l'implant chirurgical 2, et qui permettent de solidariser ce dernier aux corps osseux 3, 4.

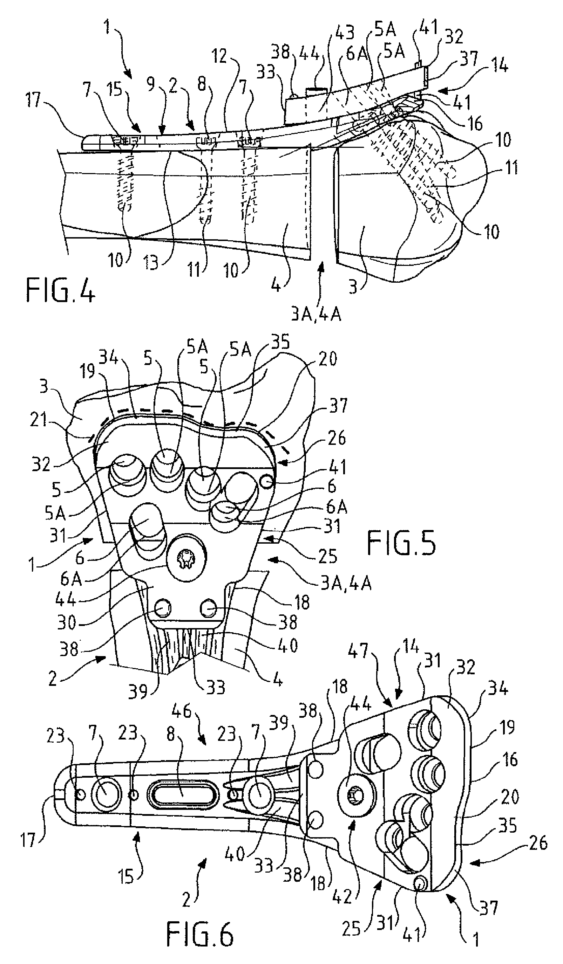

En l'espèce, et tel qu'illustré à la figure 2, un implant chirurgical 2 apte à coopérer avec le sabot 1 de l'invention, afin que ce dernier en aide le positionnement et la fixation, comporte un corps principal 9, lequel est destiné à être rapporté sur les corps osseux 3, 4. Les moyens de fixation 5, 6, 7, 8 sont préférentiellement formés par des ouvertures de vissage 5, 6, 7, 8, dans lesquelles sont destinées à être insérées des vis de fixation 10, 11 (visibles à la figure 4) pour solidariser ledit implant chirurgical 2, en particulier son corps principal 9, avec les corps osseux 3, 4 par vissage desdites vis de fixation dans ces derniers.

On entend par « *corps osseux* » 3, 4 des os, des cartilages, des fragments d'os ou de cartilage, voire des tendons, ou une combinaison de ces derniers, de préférence destinés à être fusionnés par ostéosynthèse avec l'aide de l'implant chirurgical 2 dont le sabot 1 de l'invention vient aider au positionnement et à la fixation, par exemple deux fragments d'un même os fracturés séparés par un trait de fracture 3A, 4A.

Tel qu'illustré à la figure 2, le corps principal 9 de l'implant chirurgical 2 présente avantageusement une surface antérieure 12 destinée à reposer au moins partiellement sur lesdits corps osseux 3, 4, et une surface libre 13 opposée à la surface antérieure 12 formant une surface dorsale préférentiellement libre lorsque l'implant chirurgical 2 est en place sur lesdits corps osseux 3, 4.

Dans ce qui suit, la description et les dessins se réfèrent préférentiellement à un implant chirurgical de type plaque radiale distale, destiné au traitement d'une fracture de l'extrémité distale du radius de l'avant-bras d'un patient, et donc à un sabot d'aide au positionnement et à la fixation adapté à ce type d'implant.

Toutefois, le sabot 1 de l'invention pourra être destiné à d'autres types d'implants d'ostéosynthèse et pourra donc présenter une conception différente de celle exposée ci-après. Il pourra bien évidemment en outre, et sans pour autant sortir du cadre de l'invention, être destiné à être mis en oeuvre en combinaison avec tout type d'implant 2 destiné à être rapporté sur des corps osseux 3, 4 d'un patient.

Le corps principal 9 d'un implant chirurgical 2 de type plaque radiale distale est préférentiellement formé par une plaque distale 14 qui se prolonge par une patte proximale 15.

Ladite plaque distale 14 forme une portion du corps principal 9 préférentiellement située à une première extrémité de ce dernier, la patte proximale 15 formant l'autre extrémité dans la continuité de ladite plaque distale 14. Le corps principal 9 s'étend préférentiellement longitudinalement entre :
- un bord distal 16, à partir duquel s'étend la plaque distale 14, et
- un bord proximal 17, à partir duquel s'étend la patte proximale 15, et transversalement entre deux bords latéraux 18.

La plaque distale 14 est préférentiellement conçue et conformée pour être rapportée à un corps osseux extrémal, préférentiellement distal, c'est-à-dire situé à l'avant ou à l'antérieur des corps osseux 3, 4, de préférence à une partie distale de l'os du radius, c'est-à-dire au plus près du poignet du patient.

Selon le mode de réalisation illustré aux figures, cette plaque distale 14 présente une forme sensiblement trapézoïdale, tandis que la patte proximale 15 présente une forme sensiblement allongée, de sorte que l'implant 2 présente une forme générale en spatule.

Le bord distal 16 de l'implant chirurgical 2, au positionnement et à la fixation duquel le sabot 1 de l'invention vient en aide, présente avantageusement une portion médiale 19 et une portion latérale 20, cette dernière étant en retrait par rapport à ladite portion médiale 19 de manière à pouvoir épouser la ligne anatomique des corps osseux 3, 4, en particulier un os du radius du patient.

De préférence, le bord distal 16 s'étend transversalement et est composé de deux portions successives, la portion médiale 19 étant avancée par rapport à la portion latérale 20. Le bord distal 16 présente ainsi avantageusement une forme en « S » pour limiter la douleur subie par le patient.

Epousant la ligne anatomique des corps osseux 3, 4, cette forme en « S » correspondant de fait avantageusement au tracé de la ligne de crête de la partie palmaire du radius, ou ligne « *watershed* » 21, visible à la figure 5 sous la forme d'un trait discontinu.

On entend par « *portion médiale* » la partie du bord distal 16 destinée à être disposée préférentiellement du côté médial du radius. La portion latérale 20 est préférentiellement destinée à être disposée du côté radial de l'avant-bras, opposé au côté médial.

Ainsi, l'implant chirurgical 2 sur lequel le sabot 1 de l'invention est destiné à être positionné, peut se présenter sous la forme de deux variantes principales formées par un implant pour avant-bras gauche (non représenté aux figures) et un implant pour avant-bras droit (représenté aux figures).

De façon préférentielle, le corps principal 9 de l'implant 2 est pourvu d'ouvertures de vissage distales 5, d'au moins une ouverture de vissage proximale 7, qui sont ménagées respectivement dans la plaque distale 14 et dans la patte proximale 15, et qui forment ou contribuent à former des moyens de fixation 5, 6, 7, 8 par l'intermédiaire desquels l'implant 2 maintien en position lesdits corps osseux 3, 4 les uns par rapport aux autres. Les ouvertures de vissage 5, 7 sont préférentiellement destinées à accueillir chacune une vis de fixation 10, 11 de l'implant chirurgical 2 sur les corps osseux 3, 4, tel que cela est particulièrement visible sur la figure 4.

De façon préférentielle, le corps principal 9 de l'implant chirurgical 2 est également pourvu d'un orifice 22 traversant, non destiné à recevoir une vis de fixation, ménagé au travers dudit corps principal 9 au niveau de la zone qui relie entre elles les plaque distale 14 et patte proximale 15.

Tel qu'illustré notamment à la figure 2, de façon préférentielle, l'implant chirurgical 2 comprend en outre des trous de guidage 23 traversants ménagés dans le corps principal 9, l'implant chirurgical 2 étant destiné à être enfilé par l'intermédiaire desdits trous de guidage 23 sur des broches de guidage (non représentées) lors de sa mise en place dans le corps du patient.

Le sabot 1 est prévu pour être déposé temporairement, lors de la mise en place de l'implant chirurgical 2 dans le corps du patient, sur la surface libre 13 dudit implant 2, cette surface libre 13 correspondant à une surface opposée à la surface antérieure 12 de l'implant 2, cette dernière étant destinée à reposer au moins partiellement sur lesdits corps osseux 3, 4.

Selon une caractéristique importante de l'invention, le sabot 1 comporte un corps principal 24 qui comporte lui-même au moins une zone radio-transparente 25 et au moins une zone radio-opaque 26, et des moyens de positionnement 27 dudit sabot 1 relativement à l'implant chirurgical 2 dans une position relative prédéterminée, de sorte à permettre le contrôle du positionnement dudit implant 2 relativement auxdits corps osseux 3, 4 du patient.

Le principe général de l'invention recouvre en effet un sabot 1 destiné à être rapporté sur un implant 2 dans au moins une position connue et parfaitement maitrisée, par le biais desdits moyens de positionnement 27 (qui seront explicités plus loin), ledit sabot 1 comportant au moins une zone radio-opaque 26 qui agit, lors d'un contrôle par radiographie, comme un repère visible du positionnement dudit implant 2 relativement auxdits corps osseux 3, 4, ledit repère étant ainsi avantageusement déporté et amovible de l'implant 1.

Le sabot 1 de l'invention trouve son application la plus intéressante lorsque l'implant 2 auquel il est rapporté est totalement radio-transparent. Dans ce cas de figure préférentiel, la zone radio-opaque 26 du sabot 1 constitue ainsi avantageusement le seul moyen de repère du positionnement dudit implant 2.

On n'exclut cependant pas l'association du sabot 1 de l'invention avec un implant 2 qui ne serait pas radio-transparent, mais qui, par exemple, n'aurait de radio-transparent que la zone de l'implant 2 sur laquelle est positionné le sabot 1, ou avec un implant 2 qui serait totalement radio-opaque. Dans une telle configuration extrême, la zone radio-opaque 26 du sabot 1 ne constituerait alors pas le seul, mais au moins un, moyen de repère du positionnement dudit implant 2.

Préférentiellement, le corps principal 24 du sabot 1 comporte une base 28, destinée à venir en appui sur ledit implant chirurgical 2.

La base 28 présente avantageusement une surface inférieure 29 destinée à reposer au moins partiellement sur la surface libre 13 de l'implant 2 et une surface supérieure 30, opposée et sensiblement parallèle à la surface inférieure 29, formant une surface dorsale préférentiellement libre lorsque le sabot 1 est en place sur ledit implant chirurgical 2.

Cette base 28 présente des bords latéraux 31, un bord distal 32 et un bord proximal 33. Ladite base 28 s'étend donc ainsi longitudinalement entre le bord distal 32 et le bord proximal 33, et transversalement entre deux bords latéraux 31.

Les bords latéraux 31, le bord distal 32 et le bord proximal 33 délimitent avantageusement un contour correspondant sensiblement à au moins une partie du contour dudit implant 2 sur lequel le sabot 1 est destiné à être positionné.

En particulier, selon le mode de réalisation préférentiel illustré aux figures, le sabot 1 est destiné à venir se positionner uniquement sur la plaque distale 14 de l'implant 2.

Ainsi, comme illustré à la figure 1, le bord distal 32 du sabot 1 présente avantageusement une première portion 34 et une seconde portion 35, cette dernière étant en retrait par rapport à ladite première portion 34. De préférence, le bord distal 32 s'étend transversalement et est composé de deux portions successives, la première portion 34 avancée par rapport à la seconde portion 35. Le bord distal 32 présente ainsi avantageusement une forme en « *S* ». La forme du bord distal 28 correspond donc ainsi avantageusement à la forme en « *S* » du bord distal 16 de l'implant 2.

De plus, les bords latéraux 31 sont de préférence sensiblement droits et lesdits bords latéraux 31, le bord distal 32 et le bord proximal 33 sont configurés de telle sorte que la base 28 présente un contour sensiblement trapézoïdal, correspondant ainsi au contour de la plaque distale 14 de l'implant 2.

Selon le mode de réalisation préférentiel illustré aux figures, et en particulier à la figure 1, la base 28 présente au niveau de son bord distal 32 une zone dont l'épaisseur décroit préférentiellement légèrement de la partie médiane de la base 28 vers ledit bord distal 32. En outre, toujours selon ce mode de réalisation préférentiel, le bord proximal 33 forme un décroché faisant saillie de la forme généralement trapézoïdale de ladite base 28.

Il est toutefois alternativement envisageable que le sabot 1 vienne recouvrir la totalité de la surface du corps principal 9 dudit implant 2, auquel cas la base 28 pourra, de la même façon, présenter un contour correspondant au contour spatulaire dudit implant 2.

La base 28 du sabot 1 présente avantageusement des ouvertures oblongues de perçage 5A, 6A. Lorsque le sabot 1 est mis en position sur l'implant 2, ces ouvertures oblongues de perçage 5A, 6A ont vocation d'une part et dans un premier temps, à recevoir un guide de perçage 36 (visible à la figure 3) de l'os constituant les corps osseux 3, 4, tout en limitant angulairement le positionnement dudit guide de perçage 36.

En effet, lesdites ouvertures oblongues de perçage 5A, 6A sont configurées dans leur orientation respective de sorte à n'autoriser *in fine* le perçage de l'os au moyen du guide de perçage 36 que dans une direction précise donnée.

D'autre part, et dans un second temps, une fois l'os ainsi percé selon une orientation souhaitée et le guide de perçage 36 retiré, le sabot 1 étant maintenu positionné sur l'implant 2, lesdites ouvertures oblongues de perçage 5A, 6A permettent avantageusement de guider la mise en place et le vissage par le chirurgien des vis de fixation 10, 11 de l'implant 2 sur les corps osseux 3, 4.

La mise en place de ces dites vis de fixation 10, 11 est dès lors particulièrement aisée et précise.

Tel qu'illustré à la figure 1, certaines ouvertures oblongues de perçage 5A et 6A peuvent être sécantes, afin de faciliter encore davantage la mise en oeuvre du guide de perçage 36 et les vis de fixation 10, 11 dans un champ opératoire potentiellement complexe et étroit.

De la même façon, la base 28 du sabot 1 est avantageusement pourvue de trous de guidage (non représentés) destinés à être enfilés sur des broches de guidage (non représentée).

Le corps principal 24 du sabot 1 comporte, selon l'invention, et comme introduit précédemment, au moins une zone radio-transparente 25, c'est-à-dire invisible à la radiographie, et au moins une zone radio-opaque 26, c'est-à-dire au contraire visible à la radiographie.

Selon une première variante préférée de l'invention, représentée aux figures et en particulier à la figure 1, la base 28 consiste en une pièce monobloc réalisée dans un matériau sensiblement radio-transparent, apte à former au moins en partie ladite au moins une zone radio-transparente 25 du corps principal 24.

En d'autres termes, la base 28 constitue donc une pièce monobloc réalisée dans une matière sensiblement invisible à la radiographie, par un exemple du polyéther-éther-cétone (PEEK) ou une autre matière plastique biocompatible. Une telle base 28 peut être aisément fabriquée, par exemple, par usinage ou par injection.

Ladite au moins une zone radio-opaque 26 du sabot 1 selon cette première variante est quant à elle avantageusement constituée d'un élément 37 sensiblement radio-opaque, distinct de ladite base 28 et fixé au corps principal 24.

Un tel élément 37 est préférentiellement unique et monobloc, comme retenu dans le mode de réalisation préférentiel illustré à la figure 1. Ledit élément 37 est réalisé, par exemple par usinage ou par formage, dans un matériau sensiblement visible à la radiographie, par exemple un matériau métallique à base d'acier inoxydable, de titane ou d'un autre métal ou alliage de métaux biocompatibles.

Alternativement, il est tout à fait envisageable, sans sortir du cadre de l'invention, que ladite zone radio-opaque 26 soit constituée d'une pluralité d'éléments 37 réalisés dans un matériau sensiblement visible à la radiographie.

Ledit élément radio-opaque 37 est avantageusement fixé de manière ferme et définitive à la base 28 du sabot 1, par exemple par encastrement dudit élément 37 dans la base 28 ou par collage dudit élément 37 sur la base 28 à l'aide d'un adhésif adéquat.

Cette variante préférée de l'invention présente l'intérêt d'être très simple à mettre en oeuvre, car faisant appel à une association mécanique relativement simple de matériaux couramment disponibles et dont la mise en oeuvre sécurisée dans le cadre d'une utilisation thérapeutique intracorporelle est largement éprouvée.

Dans une seconde variante de l'invention, non représentée aux figures, ladite base 28 est majoritairement réalisée dans un matériau sensiblement radio-transparent apte à former au moins en partie ladite au moins une zone radio-transparente 25 du corps principal 24 du sabot 1, ledit matériau présentant localement dans sa masse une ou plusieurs zones radio-opaques aptes à former localement au moins en partie ladite au moins une zone radio-opaque 26 du corps principal 24.

Dans ce cas, la base 28 est donc une pièce unique, monobloc, réalisée dans un matériau ou un mélange de matériaux globalement radio-transparents mais présentant une variation locale notable de ses propriétés de radio-transparence de sorte à générer localement une ou plusieurs zones identifiables à la radiographie.

Un tel matériau composite peut, par exemple, être réalisé par implantation de particules radio-opaques dans une matrice radio-transparente ou encore par modulation locale de la densité d'un matériau sensiblement radio-transparent, la ou les zones de densité plus importante présentant alors une relative radio-opacité contrastant avec les zones de plus faible densité.

L'élément 37 sensiblement radio-opaque de la variante préférée de l'invention est préférentiellement positionné au niveau du bord distal 32 de la base 25 du sabot 1. Dans ce cas préférentiel, l'élément 37 radio-opaque est donc positionné en contact avec le bord distal 32, et est ainsi par exemple positionné sur, contre ou encore dans le bord distal 32 de la base 28.

Toutefois, et sans sortir pour autant du cadre de l'invention, il est envisageable que ladite zone radio-opaque 26 ne soit pas positionnée au niveau du bord distal bord distal 32 de la base 25 du sabot 1, mais par exemple au niveau d'une autre partie du sabot 1.

De manière encore plus préférentielle, l'élément 37 sensiblement radio-opaque est positionné de sorte à épouser le contour du bord distal 32 de ladite base 28, c'est-à-dire que l'élément 37 s'intègre parfaitement dans le contour du bord distal 32 et, en particulier ne s'étend pas au-delà d'une part de la surface inférieure 29 et d'autre part de la surface supérieure 30 de la base 28.

De manière encore plus préférentielle, ledit élément 37 sensiblement radio-opaque présente un profil identique à celui du bord distal 32 de ladite base 28, et reproduit ainsi très fidèlement les variations du profil du bord distal 32.

Par conséquent, l'élément radio-opaque 37 permet, à la radiographie, l'identification nette et très précise du bord distal 32 de la base 28 du sabot 1 alors même que ladite base 28 reste invisible à la radiographie.

Toujours sans sortir du cadre de l'invention, il est bien évidemment tout aussi envisageable que ledit élément 37 sensiblement radio-opaque soit positionné et configuré d'une façon différente à celle, préférentielle, décrite ci-dessus. Ledit élément 37 sensiblement radio-opaque pourra, par exemple, présenter un profil différent de celui du bord distal 32 de ladite base 28 ou encore être positionné légèrement en avant ou au contraire en retrait, dudit bord distal 32.

Dans le mode de réalisation particulièrement avantageux représenté aux figures, l'élément radio-opaque 37 se présente sous la forme d'une plaque, ou d'un ruban, encastrée dans le bord distal 32 de la base 28 du sabot 1 de sorte à affleurer la surface dudit bord distal 32 en reproduisant très fidèlement les variations de son profil. Ledit élément radio-opaque 37 pourrait, alternativement, tout aussi bien se présenter sous la forme d'un fil encastré de la même manière.

A l'examen radiographique, lors de la prise d'un cliché de dessus, c'est-à-dire un cliché pris selon une direction perpendiculaire à la surface de l'implant 2 sur lequel est positionné le sabot 1, l'élément radio-opaque 37 apparaît, selon ce mode de réalisation préférentiel, par projection dans le plan de la radiographie, sous la forme d'un trait contrastant avec le reste des éléments visibles sur la radiographie.

Ce trait est d'autant plus visible et reconnaissable lorsque l'implant 2 est réalisé dans un matériau radio-transparent. Il apparaît en effet comme un trait noir, ou au moins plus foncé, sur la radiographie. Il reste cependant parfaitement identifiable lorsque l'implant 2 est au contraire réalisé dans un matériau radio-opaque, si tant est que le matériau radio-opaque formant l'implant 2 n'est pas strictement identique au matériau formant l'élément radio-opaque 37.

Dans l'hypothèse d'un implant 2 réalisé dans un matériau sensiblement radio-opaque, il conviendrait de retenir, pour la réalisation de la zone radio-opaque 26, un matériau de densité significativement plus faible ou plus grande, ou une méthode de réalisation permettant de conférer au matériau utilisé une densité significativement plus faible ou plus grande, que celle du matériau formant l'implant 2, de sorte à faire apparaître, par contraste le trait correspondant à la zone radio-opaque 26.

Le corps principal 24 du sabot 1 comporte également, selon l'invention, et comme introduit précédemment, des moyens de positionnement 27 dudit sabot 1 relativement à l'implant chirurgical 2 dans au moins une position relative prédéterminée.

Au sens de l'invention, ladite position relative prédéterminée correspond avantageusement à une position dans laquelle le sabot 1 est placé sur la surface libre 13 de l'implant chirurgical 2 de telle sorte que le bord distal 32 et les bords latéraux 31 de la base 28 dudit sabot 1 viennent se positionner respectivement sensiblement au droit du bord distal 16 et des bords latéraux 18 de la plaque distale 14 de l'implant chirurgical 2.

Dans cette position relative prédéterminée, les ouvertures de perçage 5A, 6A, du sabot 1 se superposent et correspondent avantageusement aux ouvertures de vissage 5, 6 de l'implant chirurgical 2 lorsque le sabot 1 est rapporté sur ce dernier.

De la même façon, dans ladite position relative prédéterminée, les trous de guidage (non représentés) du sabot 1 correspondent avantageusement aux trous de guidage 23 de l'implant chirurgical 2, de manière à ce que les trous de guidage du sabot 1 puissent être enfilés sur les broches de guidage (non représentées) en se superposant aux trous de guidage 23 de l'implant chirurgical 2 lorsque ledit sabot 1 est positionné sur ledit implant 2.

De cette façon, lorsque le sabot 1 est rapporté sur l'implant chirurgical 2, et que ce dernier est positionné à l'aide des broches de guidage (non représentées) sur les corps osseux 3, 4 du patient, il est possible d'effectuer un perçage ou pré-perçage des corps osseux 3, 4, à l'aide par exemple du guide de perçage 36, en vue de l'insertion des vis de fixation 10, 11. Le pré-perçage ou perçage étant effectué à travers les ouvertures de perçage 5A, 6A du sabot 1, l'intégrité des ouvertures de vissage 5, 6 est préservée, et l'opération de mise en place de l'implant chirurgical 2 facilitée.

Il reste cependant parfaitement envisageable, sans sortir pour autant du cadre de l'invention, que ladite position relative prédéterminée corresponde à une position qui diffère de celle présentée ci-dessus. Il est par exemple possible de prévoir que, dans ladite position relative prédéterminée, le sabot 1 soit, au contraire, placé sur la surface libre 13 de l'implant chirurgical 2 de telle sorte que le bord distal 32 et les bords latéraux 31 de la base 28 dudit sabot 1 viennent se positionner respectivement en retrait ou encore en débordement du bord distal 16 et des bords latéraux 18 de la plaque distale 14 de l'implant chirurgical 2.

Quoiqu'il en soit ladite position relative prédéterminée du sabot 1 relativement à l'implant chirurgical 2 est préférentiellement définie à l'avance, par exemple selon la nature et le type de l'implant 2 considéré, et lesdits moyens de positionnement 27 sont avantageusement conçus de manière à ce que ledit sabot 1 adopte facilement, et de préférence de manière « naturelle » et intuitive, cette position relative prédéterminée lorsqu'il est rapporté à l'implant 2. Ces moyens de positionnement 27 ne constituent en revanche pas, de préférence, en tant que tels, des moyens permettant de fixer le sabot 1 à l'implant 2 auquel il est rapporté, et sont donc distincts et indépendants de tels moyens de fixation.

De manière particulièrement avantageuse, ces moyens de positionnement 27 peuvent constituer des moyens détrompeurs, empêchant ou au moins limitant sensiblement le risque de positionnement du sabot 1 relativement à l'implant 2 dans une position autre que ladite position prédéterminée.

Afin de faciliter la mise en place du sabot 1 sur l'implant 2 dans ladite position relative prédéterminée, la surface inférieure 29 de la base 28 du sabot 1, destinée à venir en contact avec l'implant 2, est préférentiellement dotée d'au moins deux pions 38 faisant saillie à partir de la surface inférieure 29 de ladite base 28.

Selon ce mode de réalisation préférentiel, ces pions 38 forment ou contribuent ainsi avantageusement à former lesdits moyens de positionnement 27.

Comme illustré à la figure 1, ces pions 38 sont préférentiellement disposés au niveau du bord proximal 33 de la base 28, de manière symétrique de part et d'autre de l'axe longitudinal de ladite base 28.

Lesdits pions 38 viennent préférentiellement de matière avec la base 28 de sorte à former une pièce d'un seul tenant.

Ils sont avantageusement destinés à venir coopérer avec au moins deux organes de guidage longitudinaux 39, 40 préférentiellement ménagés dans la surface libre 13 de l'implant 2 afin de permettre le positionnement dudit sabot 1 relativement audit implant 2 dans ladite position relative prédéterminée.

Selon le mode de réalisation préférentiel présenté notamment à la figure 2, lesdits organes de guidage longitudinaux 39,40 sont constitués par deux sillons longitudinaux 39, 40 ménagés au niveau de la zone du corps principal 9 qui relie la plaque distale 14 à la patte proximale 15 de l'implant 2. Les sillons longitudinaux 39, 40 sont préférentiellement disposés en « V » de façon parallèle aux bords latéraux 18 de l'implant 2, l'ouverture du « *V* » étant orientée en direction du bord distal 16 de l'implant 2.

La présence de ces deux pions 38 et leur coopération avec les organes de guidage longitudinaux 39, 40 de l'implant 2, permettent avantageusement de faciliter le positionnement précis du sabot 1 sur l'implant 2, par exemple par coulissement desdits pions 38 dans lesdits organes de guidage longitudinaux 39, 40, tout en interdisant tout mouvement de rotation dudit sabot 1 sur ledit implant 2.

De préférence, lesdits moins deux pions 38 font saillie de part et d'autre des surfaces inférieure 29 et supérieure 30 de ladite base 28 de sorte à ce que le sabot 1 puisse être positionné de manière réversible sur l'implant 2 et, selon deux variantes, constituer ainsi un sabot 1 pour implant droit ou pour implant gauche.

Dans le mode de réalisation préférentiel, représenté aux figures, selon lequel l'implant 2 constitue une plaque radiale distale, le sabot 1 peut dès lors constituer un sabot 1 pour implant d'avant-bras droit, tel que représenté aux figures, ou un sabot pour implant d'avant-bras gauche, par simple retournement du sabot 1 relativement à la surface libre 13 de l'implant 2.

La surface inférieure 29 de la base 28 du sabot 1 est en outre, selon le mode de réalisation préférentiel représenté aux figures, dotée d'au moins un troisième pion 41. Faisant également saillie à partir de la surface inférieure 29 de la base 28, ce troisième pion 41 est positionné à proximité du bord distal 32 de ladite base 28, de sorte à venir en appui sur le bord distal 16 de l'implant 2 lors que le sabot 1 est en place sur ledit implant 2.

Ainsi, et en particulier dans le cas où la plaque distale 14 de l'implant 2 présente un chanfrein au niveau de son bord distal 16, ce troisième pion 41 permet avantageusement une reprise par l'implant 2 de l'effort exercé par le chirurgien sur le sabot 1, contribuant ainsi à la précision du geste de pose et de fixation de l'implant 2 ainsi qu'à la bonne tenue mécanique du sabot 1 en évitant les phénomènes de porte-à-faux.

De façon similaire à la configuration des deux pions 38, ce troisième pion 41 fait également avantageusement saillie, selon le mode de réalisation préférentiel représenté aux figures, de part et d'autre des surfaces inférieure 29 et supérieure 30 de ladite base 28 de sorte à ce que le sabot 1 puisse être positionné de manière réversible sur l'implant 2.

De préférence, le sabot 1 est conçu pour que ladite au moins une zone radio-opaque 26 du corps principal 24 présente une configuration qui reprend celle d'une zone anatomique, par exemple la ligne « *watershed »* 21 donnée en regard de laquelle ladite zone radio-opaque 26 est destinée à être positionnée.

En d'autres termes, le sabot 1 est avantageusement conçu de sorte que le profil de la zone radio-opaque 26 de sa base 28 suive fidèlement le profil d'une zone anatomique d'intérêt particulier des corps osseux 3, 4, de sorte que le chirurgien puisse contrôler précisément le positionnement de l'implant 2 en regard de cette zone anatomique par superposition, à la radiographie, du trait formé par la projection de la zone radio-opaque 26 sur le profil de zone anatomique concernée.

De manière encore plus préférentielle, selon le mode de réalisation illustré aux figures, ladite zone anatomique est la ligne « *watershed* » 21 d'un radius.

En effet, dans le mode de réalisation particulièrement avantageux illustré notamment à la figure 4, lorsque le sabot 1 est rapporté sur ledit implant chirurgical 2 et positionné dans ladite position prédestinée à l'aide des moyens de positionnement 27, la base 24 du sabot 1 est préférentiellement positionnée sur la plaque distale 14 dudit implant 2 de sorte, notamment, à ce que le bord distal 28 du sabot 1 vienne se positionner précisément au droit du bord distal 16 de l'implant 2.

Puisque ladite au moins une zone radio-opaque 26 du corps principal 24 du sabot 1 permet avantageusement l'identification nette et très précise du bord distal 32 de la base 28 du sabot 1 à la radiographie, alors, de fait, ladite au moins une zone radio-opaque 26 se trouve, par l'assemblage du sabot 1 sur l'implant 2, conformée et configurée de sorte à permettre indirectement le contrôle du positionnement dudit implant 2, et plus particulièrement de son bord distal 16, relativement à une zone anatomique donnée du corps du patient au niveau de laquelle est fixé ledit implant 2.

Selon ce mode de réalisation particulièrement avantageux, le bord distal 16 de l'implant 2 présente préférentiellement, comme décrit précédemment, une forme en « S » qui, épousant la ligne anatomique des corps osseux 3, 4, correspond de fait avantageusement au tracé de la ligne de crête de la partie palmaire du radius, ou ligne « *watershed* ».

Dès lors, par assemblage du sabot 1 sur l'implant 2 selon la position relative prédéterminée, la zone radio-opaque 26 du sabot 1 reprend avantageusement la configuration de cette zone anatomique préférentiellement considérée que constitue la ligne « *watershed* » 21, comme cela est illustré à la figure 5.

Ladite au moins une zone radio-opaque 26, préférentiellement matérialisée par cet élément radio-opaque 37, permet alors de contrôler la position de l'implant 2 relativement à la ligne « *wafershed* » 21 du radius sur lequel l'implant 2 est destiné à être fixé, comme illustré à la figure 5.

En visualisant simultanément à la radiographie, d'une part, cette ligne « *watershed* » 21 et, d'autre part, la projection dans le plan de la radiographie de la position de l'élément radio-opaque 37 qui reflète celle du bord distal 16 de l'implant 2, le chirurgien peut venir positionner de manière très précise l'implant 2 sur les corps osseux 3, 4, tout en surveillant que ledit bord distal 16 ne vient pas déborder de ladite ligne « *wafershed* » 21.

Alternativement, il est bien entendu parfaitement envisageable que, sans sortir du cadre de l'invention, la zone radio-opaque 26 de la base 28 du sabot 1 soit conformée et configurée d'une manière différente afin de reprendre la configuration d'une autre zone anatomique d'intérêt et de permettre, à l'aide du sabot 1, le positionnement précis de l'implant 2 relativement à cette autre zone anatomique.

De manière préférentielle, le corps principal 24 du sabot 1 comporte, en outre, un moyen de solidarisation 42, dans ladite position relative prédéterminée, dudit sabot 1 relativement audit implant chirurgical 2.

Ce moyen de solidarisation 42 permet avantageusement de maintenir fermement, mais de manière amovible, le sabot 1 en position sur implant chirurgical 2, de sorte à permettre le guidage précis du guide de perçage 36, sans risquer que l'effort appliqué par le chirurgien sur ledit guide de perçage 36 n'entraine malencontreusement la désolidarisation du sabot 1 et de l'implant 2.

De manière préférentielle, ce moyen de solidarisation 42 est constitué d'un orifice de solidarisation 43 traversant la base 28 du corps principal 24, ledit orifice de solidarisation 43 étant destiné à accueillir une vis de solidarisation 44 du sabot 1 sur l'implant chirurgical 2.

Selon le mode de réalisation préféré illustré aux figures, et en particulier à la figure 1, cet orifice de solidarisation 43 est ménagé dans la base 28 du sabot 1 de sorte à être plus proche du bord proximal 33 que du bord distal 32.

Ainsi, lorsque le sabot 1 est positionné sur l'implant 2 dans la position relative prédéterminée définie précédemment, ledit orifice de solidarisation 43 avantageusement vient coïncider avec l'orifice traversant 22 dudit implant 2, ledit orifice traversant 22 constituant dès lors avantageusement un élément de réception 45 destiné à coopérer avec ledit moyen de solidarisation 42 dudit sabot 1 grâce à la vis de solidarisation 44, de sorte à permettre la solidarisation du sabot 1 sur l'implant 2.

De manière encore plus préférentielle, ladite vis de solidarisation 44 est une vis qui permet le verrouillage du sabot 1 sur l'implant chirurgical (2) par une rotation de seulement un quart de tour de ladite vis 44.

Le chirurgien peut ainsi verrouiller le sabot 1 en position sur l'implant chirurgical 2 de manière particulièrement pratique, rapide et sécurisée.

Alternativement, et sans sortir du cadre de l'invention, le moyen de solidarisation 42 peut être constitué d'une vis de solidarisation 44 solidaire dudit corps principal 24, c'est-à-dire pouvant coulisser ou tourner dans ledit orifice de solidarisation 43, tout en restant en liaison mécanique avec le corps principal 24.

Cette variante, non représentée aux figures, présente l'avantage de limiter sensiblement le risque de perte de la vis de solidarisation 44, en particulier dans le corps du patient, lors du retrait du sabot 1 de l'implant 2. Toutefois, dans une telle configuration, le sabot 1 ne présente plus le caractère réversible décrit précédemment qui lui permet d'être associé, par simple retournement, à un implant 2 pour avant-bras droit ou à un implant 2 pour avant-bras gauche.

Il est également parfaitement envisageable, sans sorti du cadre de l'invention, que la vis de solidarisation 44 soit remplacée par un autre système de fixation de type quart de tour, ou encore par un système de fixation qui ne soit pas de type quart de tour.

L'invention concerne également un kit chirurgical 46, tel que notamment illustré à la figure 6, qui inclut un implant 2 chirurgical destiné à être rapporté sur des corps osseux 3, 4 d'un patient, par exemple de son avant-bras, et, d'autre part, un sabot 1 conforme à la description faite précédemment.

Préférentiellement, et conformément à l'exemple pris précédemment et illustré aux figures, l'implant 2 inclus dans le kit chirurgical 46 est un implant d'ostéosynthèse et, plus particulièrement, une plaque radiale distale, c'est-à-dire destiné au traitement d'une fracture de l'extrémité distale du radius de l'avant-bras d'un patient.

Dès lors, corrélativement, le sabot 2 du kit chirurgical 46 constitue un sabot d'aide au positionnement et à la fixation adapté à un implant d'ostéosynthèse de type une plaque radiale distale.

L'invention ne se limitant toutefois pas à un sabot 1 d'aide au positionnement et à la fixation d'un implant d'avant-bras, le kit chirurgical 46 pourra inclure par exemple un implant tibial ou un implant palmaire, et leur sabot correspondant.

Ledit implant chirurgical 2 du kit chirurgical 46 comporte, de préférence, un élément de réception 45 destiné à coopérer avec ledit moyen de solidarisation 42 dudit sabot 1 et au moins une partie radio-transparente 47, de sorte à permettre d'une part la fixation, dans ladite position relative prédéterminée, dudit sabot 1 sur ledit implant chirurgical 2, et, d'autre part, le contrôle du positionnement dudit implant 1 relativement auxdits corps osseux 3, 4 du patient.

En effet, comme introduit précédemment, le sabot 1 de l'invention trouve son application la plus intéressante lorsque l'implant 2 auquel il est rapporté est totalement radio-transparent, ou au moins en partie radio-transparent.

A ce titre, de manière encore plus préférentielle, le corps principal 9 de l'implant chirurgical 2, et plus généralement l'implant chirurgical 2, pourra former avantageusement une seule pièce monobloc et unitaire réalisée en un matériau radio-transparent, de préférence du polyéther-éther-cétone (PEEK).

De façon préférentielle, le kit chirurgical 46 inclut un sabot 1 dont la surface inférieure 29 de la base 28, destinée à venir en contact avec l'implant 2, est dotée d'au moins deux pions 38, faisant saillie à partir de la surface inférieure 29 de ladite base 28, et destinés à venir coopérer avec au moins deux organes de guidage longitudinaux 39, 40, préférentiellement formés par les sillons longitudinaux 39, 40, ménagés à la surface dudit implant 2 afin de permettre le positionnement dudit sabot 1 relativement audit implant 2 dans ladite position relative prédéterminée.

Le kit chirurgical 46 inclut, de manière encore plus préférentielle, le sabot 1 précédemment décrit, dans sa variante selon laquelle la base 28 dudit sabot 1 comporte au moins deux pions 38 qui font saillie de part et d'autre des surfaces inférieure 29 et supérieure 30 de ladite base 28, de sorte à ce que le sabot 1 puisse être positionné de manière réversible et constituer ainsi un sabot pour implant gauche ou un sabot pour implant droit.

De la même façon, l'implant 1 inclus dans le kit chirurgical 46 se présente avantageusement selon sa variante préférée dans laquelle sa base 28 comporte le troisième pion 4, celui-ci faisant également avantageusement saillie, selon le mode de réalisation préférentiel représenté aux figures, de part et d'autre des surfaces inférieure 29 et supérieure 30 de ladite base 28.

Ceci permet avantageusement de simplifier la gestion des stocks et la préparation des kits chirurgicaux à commercialiser, puisqu'il n'y a dès lors qu'une seule référence de sabot 1 à prendre en compte pour chacune des variantes droite ou gauche d'un implant 2.

Lorsque le sabot 1 de l'invention est rapporté sur l'implant chirurgical 2 du kit chirurgical 46 dans ladite position relative prédéterminée, ladite au moins une zone radio-transparente 25 du corps principal 24 du sabot 1 est avantageusement destinée à venir en regard d'au moins une partie radio-transparente 47 dudit implant 2.

De manière encore plus préférentielle, dans ladite position relative prédéterminée du sabot 1 sur l'implant 2, le bord distal 32 de la base 28 du sabot 1 est destiné à venir sensiblement en regard d'une extrémité distale dudit implant chirurgical 2.

Dans le mode de réalisation préférentiel, illustré aux figures, selon lequel l'implant 2 est une plaque radiale distale, le bord distal 32 de la base 28 du sabot 1 vient donc se positionner en regard de la plaque distale 14 dudit implant 2, préférentiellement au droit du bord distal 16 de l'implant 2.

Ainsi, lesdits sabot 1 et implant 2 du kit chirurgical 46 se trouvent préférentiellement configurés l'un par rapport à l'autre de sorte à ce que le sabot 1 trouve son application la plus avantageuse, à savoir qu'il constitue grâce à sa zone radio-opaque 26 un repère déporté particulièrement utile de la position précise de l'implant 2 et, plus précisément du bord distal 16 de l'implant 2, relativement aux corps osseux 3, 4 du patient.

Conformément à la description préférentielle précédemment faite desdits sabot 1 et implant 2, lorsque ledit sabot 1 du kit 46 est rapporté sur un implant 2 chirurgical dudit kit 46, ladite au moins une zone radio-opaque 26 du corps principal 24 du sabot 1 est avantageusement conformée et configurée de sorte à permettre le contrôle du positionnement dudit implant 2 relativement à une zone anatomique donnée, et plus particulièrement, relativement à la ligne « *watershed* » 21 d'un radius, la configuration de ladite ligne « *watershed »* 21 étant avantageusement reprise par la zone radio-opaque 34, pour les raisons exposées précédemment.

En outre, de manière préférentielle, ledit élément de réception 45 dudit implant 2 est constitué d'un orifice 48 destiné à coopérer avec ladite vis de solidarisation 41 dudit sabot 1 afin de permettre le verrouillage dudit sabot 1 relativement audit implant 2 dans ladite position relative prédéterminée, ledit orifice 48 n'étant par ailleurs pas destiné à la fixation dudit implant sur lesdits corps osseux 3, 4.

Dans un mode de réalisation préférentiel de l'invention, cet élément de réception 45 est constitué de l'orifice traversant 22 de l'implant 2 conforme à la description préférentielle qui en a été faite précédemment.

En conséquence, l'association du sabot 1 de l'invention avec un implant chirurgical 2 conforme à la description préférentielle qui en est faite plus haut, et qui est illustrée aux figures, constitue un kit chirurgical 46 particulièrement pratique d'utilisation pour le chirurgien et qui lui permet de répondre pleinement à la problématique de la maitrise du positionnement précis du bord distal 16 de l'implant 2 relativement à la ligne *« watershed »* 21 du radius traité dans le cas particulier où l'implant 2 est radio-transparent ou lorsque au moins sa plaque distale 14 est radio-transparente.

De manière particulièrement avantageuse, le kit chirurgical 46 pourra également inclure les vis de fixation 10, 11 du sabot 1 sur l'implant 2, des broches de guidage, et la vis 44 de solidarisation du sabot 1 en position sur ledit implant 2, le tout étant préférentiellement conditionné sous emballage stérile.

De plus, afin de faciliter l'intervention du chirurgien et, notamment de réduire la durée de l'opération de pose et de fixation de l'implant 2 dans le corps du patient, le kit chirurgical 46 pourra encore plus avantageusement inclure le sabot 1 de l'invention déjà positionné et solidarisé sur ledit implant 2 selon ladite position relative prédéterminée.

L'invention pourra enfin concerner en tant que tel un procédé de mise en place d'un implant chirurgical 2 à l'aide du sabot 1 du kit chirurgical 46 décrit ci-avant. La description qui précède concernant le sabot 1, l'implant chirurgical 2 et le kit chirurgical 46 est également valable pour le procédé de mise en place explicité ci-après.

Le procédé de mise en place de l'implant chirurgical 2 à l'aide du sabot 1 de l'invention comprend avantageusement au moins les étapes successives suivantes :
- on incise le corps du patient afin de pouvoir introduire l'implant chirurgical 2 à l'intérieur, au niveau des corps osseux 3, 4 à traiter,
- on implante des broches de guidage dans les corps osseux 3, 4, par exemple par impaction, et optionnellement avec l'aide de l'implant chirurgical 2 ou du sabot 1, les broches de guidage (non représentées) pouvant être impactées dans les corps osseux 3, 4 au travers des trous de guidage 22 de ces derniers,
- on positionne l'implant chirurgical 2 sur les corps osseux 3, 4, à l'aide des broches de guidage, de façon à ce que la surface antérieure 12 de l'implant 2 repose au moins partiellement sur les corps osseux 3, 4, la plaque distale 14 de l'implant 2 étant en contact avec une partie extrémale de l'os à traiter formant le corps osseux 3 et la patte proximale 15 étant en contact avec une partie intermédiaire ou médiane de l'os à traiter formant le corps osseux 4,
- on rapporte le sabot 1 sur la surface libre 13 de l'implant chirurgical 2, en faisant préférentiellement coulisser les pions 38 dans les organes de guidage longitudinaux 39, 40 ménagés dans ladite surface libre 13 de l'implant chirurgical 2, de sorte à venir mettre en regard le bord distal 32 de la base 28 du sabot 1 avec le bord distal 16 de la plaque distale 14 de l'implant 2 et les ouvertures de vissage distales 5, 6 de ladite plaque distale 14 avec les ouvertures de perçage 5A, 6A du sabot 1,
- on solidarise le sabot 1 en position sur l'implant chirurgical 2 à l'aide de la vis de solidarisation 44,
- on effectue une radiographie pour vérifier, par visualisation de la zone radio-opaque 26 du sabot 1, le bon positionnement de l'implant chirurgical 2 sur les corps osseux 3, 4, relativement à la zone de fracture 3A, 4A à traiter et à son environnement anatomique,
- on effectue un pré-perçage des corps osseux 3, 4 au travers de tout ou partie des ouvertures de perçage 5A, 6A du sabot 1 et à travers les ouvertures 7, 8 de la patte proximale 15 de l'implant 2,
- on insère une première vis de fixation 10 de serrage dans l'une des ouvertures de vissage distales 5 de la plaque distale 14 de l'implant 2, et une deuxième vis de fixation 11 de serrage dans l'ouverture de vissage 8 de la patte proximale 15,
- avant de serrer lesdites première et deuxième vis de fixation de serrage 10, 11, on règle la proximité et l'orientation des corps osseux 3, 4 en faisant coulisser la deuxième vis de fixation 11 le long de l'ouverture de vissage 8,
- on serre la première et la deuxième vis de fixation de serrage 10, 11,
- on insère ensuite les vis de fixation 9 dans tout ou partie des ouvertures de vissage 5, 6, 7 restantes, afin d'assurer une fixation durable de l'implant chirurgical 2 dans le corps du patient,
- au fur et à mesure des opérations d'insertion et de serrage des vis de fixation 10, 11, on retire les broches de guidage des corps osseux 3, 4,
- on effectue une radiographie pour vérifier l'orientation des vis 10, 11 à travers les corps osseux 3, 4,
- on déverrouille et retire le sabot 1 de l'implant chirurgical 2,
- on referme l'incision du corps du patient.

Bien entendu, certaines étapes pourront être inversées sans sortir du cadre de l'invention. En particulier, l'étape d'assemblage du sabot 1 sur l'implant 2 peut être réalisée préalablement à l'insertion dudit implant 2 dans le corps du patient, de sorte à faciliter le travail du chirurgien.

En définitive, le sabot 1 permet une mise en place aisée, précise et rapide de l'implant chirurgical 2, contribuant ainsi à permettre une ostéosynthèse particulièrement rapide des corps osseux 3, 4, et de bonne qualité, tout en limitant les complications post-opératoires et la douleur subie par le patient.

## Revendications

1. Sabot (1) d'aide au positionnement et à la fixation d'un implant chirurgical (2) destiné à être rapporté sur des corps osseux (3, 4) d'un patient, par exemple de son avant-bras, ledit sabot (1) comportant un corps principal (24) et étant **caractérisé en ce que** ledit corps principal (24) comporte :
- au moins une zone radio-transparente (25) et au moins une zone radio-opaque (26), et
- des moyens de positionnement (27) dudit sabot (1) relativement à l'implant chirurgical (2) dans une position relative prédéterminée,
de sorte à permettre le contrôle du positionnement dudit implant (2) relativement auxdits corps osseux (3, 4) du patient.

2. Sabot (1) selon la revendication précédente, **caractérisé en ce que** le corps principal (24) comporte une base (28), destinée à venir en appui sur ledit implant chirurgical (2), et présentant des bords latéraux (31), un bord distal (32) et un bord proximal (33) qui délimitent un contour correspondant sensiblement à au moins une partie du contour dudit implant (2).

3. Sabot (1) selon la revendication 2, **caractérisé en ce que** ladite base (28) consiste en une pièce monobloc réalisée dans un matériau sensiblement radio-transparent, apte à former au moins en partie ladite au moins une zone radio-transparente (25) du corps principal (24), ladite au moins une zone radio-opaque (26) étant constituée d'un élément (37) sensiblement radio-opaque, distinct de ladite base (28), et fixé au corps principal (24).

4. Sabot (1) selon la revendication 3, **caractérisé en ce que** ledit élément (37) sensiblement radio-opaque est disposé au niveau du bord distal (32) de ladite base (28).

5. Sabot (1) selon la revendication précédente, **caractérisé en ce que** ledit élément (37) sensiblement radio-opaque épouse le contour du bord distal (32) de ladite base (28).

6. Sabot (1) selon la revendication précédente, **caractérisé en ce que** ledit élément (37) sensiblement radio-opaque présente un profil identique à celui du bord distal (32) de ladite base (28).

7. Sabot (1) selon l'une quelconque des revendications précédentes, **caractérisé en ce que** ladite au moins une zone radio-opaque (26) du corps principal (24) présente une configuration qui reprend celle d'une zone anatomique donnée en regard de laquelle ladite zone radio-opaque (26) est destinée à être positionnée, laquelle configuration reprend, de préférence, celle de la ligne « *watershed »* (21) d'un radius.

8. Sabot (1) selon l'une quelconque des revendications précédentes, **caractérisé en ce que** le corps principal (24) comporte, en outre, un moyen de solidarisation (42), dans ladite position relative prédéterminée, dudit sabot (1) relativement audit implant chirurgical (2).

9. Kit chirurgical (46) incluant un implant chirurgical (2) destiné à être rapporté sur des corps osseux (3, 4) d'un patient, par exemple de son avant-bras, ainsi qu'un sabot (1) selon l'une des revendications précédentes.

10. Kit chirurgical (46) selon la revendication précédente, **caractérisé en ce que** l'implant chirurgical (2) comporte un élément de réception (45) destiné à coopérer avec ledit moyen de solidarisation (42) dudit sabot (1) et au moins une partie radio-transparente (47), de sorte à permettre d'une part la fixation, dans ladite position relative prédéterminée, dudit sabot (1) sur ledit implant chirurgical (2), et, d'autre part, le contrôle du positionnement dudit implant (2) relativement auxdits corps osseux (3, 4) du patient.

11. Kit chirurgical (46) selon la revendication précédente, **caractérisé en ce que**, ledit sabot (1) étant rapporté sur ledit implant chirurgical (2) dans ladite position relative prédéterminée, ladite au moins une zone radio-transparente (25) du corps principal (24) vient en regard d'au moins une partie radio-transparente (47) dudit implant (2).

12. Kit chirurgical (46) selon la revendication précédente, **caractérisé en ce que** le bord distal (32) de la base (28) du sabot (1) vient sensiblement en regard d'une extrémité distale (14) dudit implant chirurgical (2).

13. Kit chirurgical (46) selon l'une quelconque des revendications 9 à 12, **caractérisé en ce que** la surface inférieure (29) de la base (28) du sabot (1), destinée à venir en contact avec l'implant (2), est dotée d'au moins deux pions (38), faisant saillie à partir de la surface inférieure (29) de ladite base (28), et destinés à venir coopérer avec au moins deux organes de guidage (39, 40) longitudinaux ménagés dans la surface (13) dudit implant (2) afin de permettre le positionnement dudit sabot (1) relativement audit implant (2) dans ladite position relative prédéterminée.

14. Kit chirurgical (46) selon la revendication précédente, **caractérisé en ce que** lesdits au moins deux pions (38) font saillie de part et d'autre des surfaces inférieure (29) et supérieure (30) de ladite base (28), de sorte à ce que le sabot (1) puisse être positionné de manière réversible et constituer ainsi un sabot pour implant gauche ou un sabot pour implant droit.

15. Kit chirurgical (46) selon l'une quelconque des revendications 9 à 14, **caractérisé en ce que** ledit implant chirurgical (2) est une plaque radiale distale.

16. Kit chirurgical (46) selon la revendication précédente, **caractérisé en ce que**, ledit sabot (1) étant rapporté sur un implant chirurgical (2), ladite au moins une zone radio-opaque (26) du corps principal (24) est conformée et configurée de sorte à permettre le contrôle du positionnement dudit implant (2) relativement à la ligne *« watershed* » (21) d'un radius, dont la configuration est reprise par la zone radio-opaque (26).
